# EUROPÄISCHE PATENTSCHRIFT

(11) **EP 0 491 390 B1**
(45) Veröffentlichungstag und Bekanntmachung des Hinweises auf die Patenterteilung: **27.11.1996**
(21) Anmeldenummer: 91121733.9
(22) Anmeldetag: 18.12.1991
(51) Int. Cl.: A61F 13/15

(54) **Wegwerfkleidungsstück**
Disposable garment
Vêtement jetable

(30) Priorität: 18.12.1990 JP 411432/90
(43) Veröffentlichungstag der Anmeldung: 24.06.1992
(73) Patentinhaber: UNI-CHARM CORPORATION, Kawanoe-shi Ehime-ken (JP)
(72) Erfinder: Igaue, Takamitsu, Kawanoe-shi, Ehime-ken (JP); Mukai, Hirotomo, Kawanoe-shi, Ehime-ken (JP); Matsushita, Michiyo, Iyomishima-shi, Ehime-ken (JP)
(74) Vertreter: Sperling, Rüdiger, Dipl.-Ing.

(56) Entgegenhaltungen:
- EP-A- 0 219 326
- EP-A- 0 346 477
- GB-A- 2 161 059

## Beschreibung

### Hintergrund der Erfindung.

Die vorliegende Erfindung betrifft Wegwerfkleidung, die dazu benutzt wird, Körperflüssigkeiten oder Ausscheidungen aufzufangen, genauer gesagt, solche Wegwerfkleidungsstücke, die mit einer Vielzahl von Klappen ausgerüstet sind, die sich an einander gegenüberliegenden Seiten des Kleidungsstückes in Längsrichtung erstrecken und üblicherweise durch ihre eigene elastisches Schrumpfeigenschaft aufgerichtet werden, um aufrecht zu stehen.

In der US-PS 4 695 278 (nachfolgend als erster Stand der Technik bezeichnet) ist eine Wegwerfwindel offenbart, die folgende Merkmale aufweist: Eine flüssigkeitsdurchlässige innere Lage, eine flüssigkeitsundurchlässige äußere Lage, einen Flüssigkeitssaugkern, der zwischen diesen beiden genannten Lagen sandwichartig angeordnet ist, ein Paar Grundklappen (d.h. Abdichtaufschläge) die sich von seitlich einander gegenüberliegenden Seitenrändern des Kerns nach außen erstrecken und jeweils mit elastischen Teilen versehen sind und ein zweites Paar von Klappen, d.h. Sperraufschlägen, die einen Rand aufweisen, der auf Flächen der jeweiligen Basisklappen wurzelt und sich von den jeweiligen elastischen Teilen nach innen erstrecken und durch elastische Teile entlang der freien Ränder der jeweiligen zweiten Klappen aufgerichtet werden, um entlang der Wurzelränder aufrecht zu stehen. Diese Basisklappen werden gegen die jeweiligen Schenkel des Trägers gepreßt. Das zweite Klappenpaar ist an in Längsrichtung einander gegenüberliegenden Enden mit den Enden verbunden, die nach innen geklappt sind und sie sind mit mittleren Abschnitten durch ihr eigenes elastisches Zusammenziehpotential aufgerichtet, wobei die freien Ränder dieser Zwischenabschnitte gegen die Schenkelwurzel des Trägers angedrückt sind.

Die US-PS4 904 251 (nachfolgend als zweiter Stand der Technik bezeichnet) offenbart Wegwerfwindeln mit einer flüssigkeitsdurchlässigen Innenlage, einer flüssigkeitsundurchlässigen äußeren Lage, einem Saugkern, der zwischen beiden Lagen sandwichartig eingelegt ist, ein Paar Basisklappen, die sich von seitlich aneinander gegenüberliegenden Rändern des Saugkerns nach außen erstrecken und einem zweiten Klappenpaar, das Ränder aufweist, die auf der Oberfläche der jeweiligen Basisklappen wurzeln und durch elastische Teile, die entlang ihrer freien Ränder vorgesehen sind, normal aufgerichtet sind und entlang der jeweiligen Wurzelränder aufrecht stehen. Das zweite Klappenpaar ist an in Längsrichtung einander gegenüberliegenden Enden mit den Enden verbunden, die nach außen geklappt sind, wobei Zwischenabschnitte durch ihre eigene Elastizität aufgerichtet werden, wobei die freien Ränder dieser Zwischenabschnitte gegen die Wurzeln der Schenkel des Trägers gedrückt werden.

Zunächst wird der erste Stand der Technik diskutiert.

Die genannten Basisklappen haben eine recht planare Konfiguration und werden unter der Wirkung ihres eigenen elastischen Schrumpfpotentials gekrümmt, jedoch nicht ausreichend genug, um um die Schenkel in Paßform zu kommen und um zu verhindern, daß die Ausscheidungen auslaufen, so wie es üblicherweise der Fall war bei Klappen an konventionellen Wegwerfwindeln. Damit die Taschen, die durch das zweite Klappenpaar, das angepaßt ist, sich gegen die Wurzeln der Schenkel des Trägers anzulegen, adäquat geöffnet werden und die Ausscheidungen zuverlässig aufnehmen können, ist es wesentlich, daß die freien Ränder der jeweiligen zweiten Klappen stets gegen die Schenkelwurzeln stabilisiert werden. Der erste Stand der Technik offenbart weder eine Breitenabmessung, noch eine Steifigkeitseinstellung für jede zweite Klappe, die notwendig wäre, um ein ideales Anpassen zu erreichen. Die freien Enden der zweiten Klappen müssen jeweils gegen die Wurzeln der Schenkel stabilisiert bleiben, wie oben erwähnt wurde, jedoch ist der Druck, mit welchem die zweiten Klappen gegen die Schenkelwurzeln gedrückt werden, zu hoch, so daß das Tragen dieses bekannten Gegenstands für eine relativ lange Zeit zu Druckstellen auf der Haut führt und Schmerzen verursacht.

Im folgenden wird der zweite Stand der Technik betrachtet.

Die gegenannten Seitenklappen haben keine elastische Schrumpffähigkeit und können somit nicht elastisch gegen die Schenkel des Trägers gepreßt werden, um erfolgreich ein Auslaufen der Ausscheidungen zu verhindern. Zwar hat das zweite Klappenpaar eine solche Funktion, aber im Hinblick auf die Stelle, an welcher jede zweite Klappe gegen den Körper des Trägers gepreßt wird, entsprechen diese Klappen den Grundklappen des ersten Stands der Technik. Mit anderen Worten, der zweite Stand der Technik hat keine Elemente, die den zweiten Klappen des ersten Stands der Technik entsprechen.

Die Aufgabe der vorliegenden Erfindung besteht darin, ein Wegwerfkleidungsstück so zu verbessern, daß das oben erwähnte Problem, das bei den Gegenständen des ersten und zweiten Stands der Technik auftritt, durch Elemente gelöst wird, die den zweiten Klappen des ersten und des zweiten Stands der Technik entsprechen, wobei die Zugfestigkeit, die Breite und die Steifheit der Teile, die den zweiten Klappen des ersten Stands der Technik entsprechen, auf optimales Niveau gesetzt werden.

### Kurzbeschreibung der Erfindung

Die Aufgabe wird erfindungsgemäß bei einem Kleidungsstück mit einem flüssigkeitsabsorbierenden Kern, einem ersten Paar von Klappen, die sich entlang seitlich einander gegenüberliegender Seiten des Gegenstands erstrecken und einem zweiten Paar von Klappen, die sich innerhalb der jeweiligen ersten Klappen erstrecken, wobei beide, die ersten und die zweiten Klappenpaare elastische Zusammenzieheigenschaften aufweisen, durch welche diese Klappen aufgerichtet werden. Zusätzlich wird eine Zugspannung der zweiten Klappen so eingerichtet, daß sie geringer ist als eine Zugspannung der ersten Klappe und die Breite jeder zweiten Klappe ist so bemessen, daß sie größer ist als die Breite jeder ersten Klappe und die Steifigkeit jeder zweiten Klappe wird so eingestellt, daß sie niedriger ist als die Steifigkeit der ersten Klappe. Vorzugsweise zweigen das erste und das zweite Klappenpaar von der zugeordneten Basisklappe ab, die sich jeweils von den seitlich einander gegenüberliegenden Seitenrändern jedes Flüssigkeitssaugkerns nach außen erstrecken. In diesem Ausführungsbeispiel kann das erste und das zweite Klappenpaar voneinander unabhängig oder kontinuierlich ausgebildet sein.

Wenn das erfindungsgemäße Kleidungsstück mit den oben beschriebenen Merkmalen angelegt wird, werden die freien Ränder der jeweiligen ersten Klappen relativ eng an die Schenkel angepreßt, wohingegen die freien Ränder der zweiten Klappen ziemlich locker um die Schenkelwurzeln des Trägers anliegen. Während der Bewegung der Beine bewegen sich die freien Ränder der ersten Klappen gleitend auf der Haut der Schenkel und die freien Ränder der zweiten Klappen verbleiben im wesentlichen um die Schenkelwurzel festgelegt.

Erfindungsgemäß ist die Zugfestigkeit jeder zweiten Klappe geringer als diejenige der ersten Klappe, so daß selbst, wenn die zweite Klappe gegen die Haut des Trägers für eine relativ lange Zeit angepreßt bleibt, die Fläche der Haut, gegen welche die zweite Klappe gepreßt wurde, frei von jeglicher Druckstelle oder von Schmerzen ist.

Das andere wichtige Merkmal der Erfindung, nämlich daß die Breite oder Höhe jeder zweiten Seitenklappe, gemessen von dem Wurzelrand bis zum freien Ende dieser Klappe größer dimensioniert ist als jede erste Seitenklappe, gemessen von der Wurzelkante bis zum freien Rand dieser ersten Klappe und die Steifigkeit jeder zweiten Klappe, die so eingestellt ist, daß sie geringer ist als diejenige jeder ersten Klappe, bewirkt, daß jede Bewegung der Windel, des Körpers oder der Schenkel des Trägers absorbiert oder abgeschwächt wird, anstatt daß eine solche Bewegung auf die freien Ränder jeder zweiten Klappe übertragen wird, welche gegen den Wurzelbereich des Schenkels gepreßt gehalten wird.

Demzufolge wird die freie Kante jeder zweiten Klappe gegen die Wurzel stabil und daher die von der zweiten Klappe gebildete gebildete Tasche offen gehalten, so daß die Flüssigkeit oder feste Ausscheidung zuverlässig durch diese Tasche aufgenommen werden kann. Jede Menge an Ausscheidungen, die aufgrund welcher Ursache auch immer über die zweite Klappe hinausdringt, wird sicher durch die zugeordnete erste Klappe vor einem weiteren Ausfluß bewahrt.

Erfindungsgemäß wird darüberhinaus an jeder Basisklappe zwischen dem ersten und zweiten Klappenpaar, das dieser Basisklappe zugeordnet ist, eine absorbierende Lage vorgesehen, so daß jede Ausscheidungsmenge, die gelegentlich zwischen der ersten und zweiten Klappe anzutreffen ist, von dieser Sauglage aufgenommen wird, um zuverlässig ein weiteres Ausfließen zu verhindern.

### Kurze Beschreibung der Zeichnungen.

In den Figuren zeigen
- Fig. 1: eine perspektivische Ansicht einer einzelnen Wegwerfwindel in zusammengebautem Zustand als ein Ausführungsbeispiel eines Kleidungsstücks, wie es gemäß der vorliegenden Erfindung hergestellt ist;
- Fig. 2: eine perspektivische Ansicht der in Fig. 1 dargestellten Windel in aufgeklapptem Zustand;
- Fig. 3: eine Schnittansicht eines wesentlichen Teils der genannten Windel;
- Fig. 4: eine Ansicht ähnlich derjenigen in Fig. 3 eines anderen Ausführungsbeispiel;
- Fig. 5 und 6: Ansichten ähnlich derjenigen in den Figuren 3 und 4 von weiteren Ausführungsbeispielen;
- Fig. 8 und 6: ähnliche Ansichten wie in Fig. 3 und 4, die weitere Ausführuangsformen zeigen, wobei beide Ausführungsbeispiele Sauglagen auf den Basisklappen zeigen.

### Bevorzugte Ausführungsbeispiele der Erfindung.

Nachfolgend werden verschiedene Ausführungsbeispiele einer Wegwerfwindel, die in Übereinstimmung mit den Lehren der vorliegenden Erfindung hergestellt worden sind, unter Bezugnahme auf die Zeichnungen näher beschrieben.

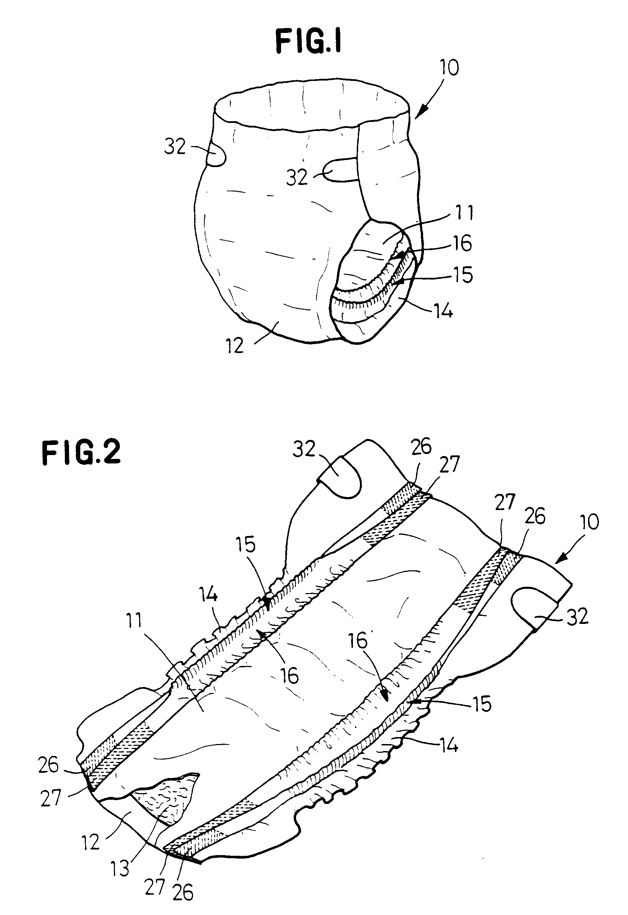

Es wird zunächst auf die Fig. 1 bis 3 bezug genommen. Die darin dargestellte Windel 10 weist eine flüssigkeitsdurchlässige innere Lage 11, eine flüssigkeitsundurchlässige äußere Lage 12, einen Flüssigkeitssaugkern 13, ein Paar seitlicher, einander gegenüberliegender Basisklappen 14, ein erstes Paar seitlicher, einander gegenüberliegender Klappen 15 und ein zweites Paar seitlicher, einander gegenüberliegender Klappen 16 auf. Die Basisklappen 14 erstrecken sich von einander gegenüberliegenden Rändern des Kerns 13 nach außen, wobei jede Abschnitte 17 und 18 der inneren und äußeren Lagen besitzt, die mittels eines Adhesivs oder mittels Schweißens (einschl. Ultraschall-Schweißens) miteinander verbunden sind.

Sowohl die ersten Klappen 15 als auch die zweiten Klappen 16 werden von einer luftdurchlässigen, jedoch einer flüssigkeitsfesten Lage 19 gebildet, die im wesentlichen über Zentralbereiche mit der oberen Fläche der jeweiligen Basisplatte 14 mittels eines Klebstoffs oder mittels Schweißens (einschl. Ultraschall-Schweißens) verbunden ist. Jede dieser ersten Klappen 15 weist ein elastisches Teil 21 in einer hülsenförmigen freien Kante 20 auf, die von einem Abschnitt der Lage 19 gebildet wird. Dieses elastische Teil 21 wurde in einem in Längsrichtung gestreckten Zustand eingebaut, so daß eine elastische Zusammenziehfähigkeit des elastischen Teils 21 diese erste Klappe 15 aufrichtet, und diese dann entlang ihrer Länge auf ihrer Wurzelkante 22, entlang welcher sie von der zugeordneten Basisklappe 15 abzweigt, aufrechtsteht.

Gleichzeitig enthält jede der Seitenklappen 16 ein elastisches Teil 24 in einer hülsenförmigen freien Kante 23, die durch einen anderen Abschnitt der Lage 19 gebildet wird. Dieses elastische Teil 24 wurde in in Längsrichtung gedehntem Zustand eingebaut, so daß eine elastische Zusammenziehfähigkeit diese zweite Klappe 16 aufrichtet und sie entlang ihrer Wurzelkante 25, welche nach innen einen Abstand zur Wurzelkante 22 besitzt, aufrecht steht. Entlang dieser Wurzelkante 25 zweigt die zweite Klappe 16 von der zugeordneten Basisklappe 14 ab.

Jede der ersten Klappen 15 ist an in Längsrichtung einander gegenüberliegenden Enden 26 auf der zugeordneten Basisklappe 14 mittels eines Adhesivs oder durch eine Schweißung (einschl. Ultraschall-Schweißung) mit einem sich in Längsrichtung erstreckenden Zwischenabschnitt dieser Klappe 15 verbunden, während dieser aufrecht steht und mit den genannten in Längsrichtung einander gegenüberliegenden Enden 26 nach innen geklappt. Es versteht sich jedoch, daß die erste Klappe 15 auf der zugeordneten Basisklappe 14 in anderer Weise befestrigt sein kann, d.h. mit dem sich in Längsrichtung erstreckenden Zwischenabschnitt in aufgerichteter Stellung gehalten, aber mit den sich in Längsrichtung gegenüberliegenden Enden 26 nach außen geklappt.

Gleichzeitig ist jede der zweiten Klappen 16 an den in Längsrichtung einander gegenüberliegenden Enden 27 auf der zugeordneten Basisklappe 14 und dem Kern 15 mittels eines Klebstoffs oder durch Schweißen (einschl. Ultraschall-Schweißen) mit einem sich in Längsrichtung erstreckenden Zwischenabschnitt verbunden, während dieser in aufrechter Stellung gehalten ist und mit den sich in Längsrichtung gegenüberliegenden Enden 27 nach innen geklappt, so daß eine relativ tiefe Tasche 13 gebildet ist.

Obwohl sowohl die ersten als auch die zweiten Klappen 15,16 die Zwischenabschnitte zwischen den sich jeweils in Längsrichtung einander gegenüberliegenden Enden 26,27 besitzen, welche normalerweise aufgerichtet sind, klappen diese Zwischenabschnitte ebenfalls nach innen, wenn die Klappen 15,16 in Längsrichtung im wesentlichen bis zur vorbestimmten Dehngrenze gestreckt werden.

Wenn die erste Klappe 15 an den in Längsrichtung einander gegenüberliegenden Enden 26 auf die zugeordnete Grundplatte 14 in der genannten alternativen Weise festgelegt wird, d.h. mit dem Zwischenabschnitt in aufgerichteter Stellung und mit den in Längsrichtung einander gegenüberliegenden Enden 26 nach außen geklappt, wird der Zwischenabschnitt offensichtlich nach außen geklappt, wenn diese Klappe 15 in ihrer Längsrichtung im wesentlichen bis zum vorbestimmten Dehnlimit gestreckt wird.

Die Zugspannung der zweiten Klappe 16 wird so eingestellt, daß sie geringer ist als die Zugspannung der ersten Klappe 15, damit der Flächenbereich auf der Haut des Trägers gegen den die zweite Klappe 16 unter dem Zusammenziehpotential der zweiten Klappe gepreßt wird, frei ist von jeglicher Druckstelle oder von jeglichem Schmerz, wenn die Klappe 16 gegen die Haut für eine relativ längere Zeit angepreßt bleibt.

Eine Zugspannung des elastischen Teils 21 wird üblicherweise auf einen Wert von 100g bis 300g eingestellt. Eine Breite (oder Höhe) W₂ der zweiten Klappe 16, wenn sie von der Wurzelkante 25 bis zur freien Kante 29 gemessen wird,ist so dimensioniert, daß sie größer ist als die Breite (oder Höhe) W₁ der ersten Klappe 15, gemessen von der Wurzelkante 22 bis zur freien Kante 28. Die Steifigkeit der zweiten Klappe 16 wird so eingestellt, daß sie geringer ist als die Steifigkeit der ersten Klappe 15, so daß jegliche Bewegung der Windel 10, des Körpers oder der Schenkel des Trägers absorbiert oder abgeschwächt wird, anstatt auf die freie Kante 29 der zweiten Kante 16 übertragen zu werden, welche um den Schenkel bzw. die Schrittfalte gehalten wird.

Beispielsweise beträgt die Breite W₁ der ersten Klappe 15 zwischen 20 mm bis 35 mm, die Breite W₂ der zweiten Klappe 16 zwischen 30 mm und 100 mm und die Längs- und Querabmessungen der zweiten Klappe 16 gemessen in Übereinstimmung mit dem japanischen Industriestandard P8143 kann weniger als 100 mm bzw. weniger als 60 mm betragen. Die entsprechenden Abmessungen der ersten Klappe 15 können im wesentlichen gleich jenen der Seitenklappen in üblichen Wegwerfwindeln nach dem Stand der Technik sein. Vorzugsweise sollte die Breite W₃, gemessen zwischen den Wurzelkanten 22,25 nicht größer sein als die Breite W₁ der ersten Klappe 15. Die Wurzelkante 25 kann neben oder in Kontakt mit der zugeordneten äußeren Seitenkante des Saugkerns 13 angeordnet sein.

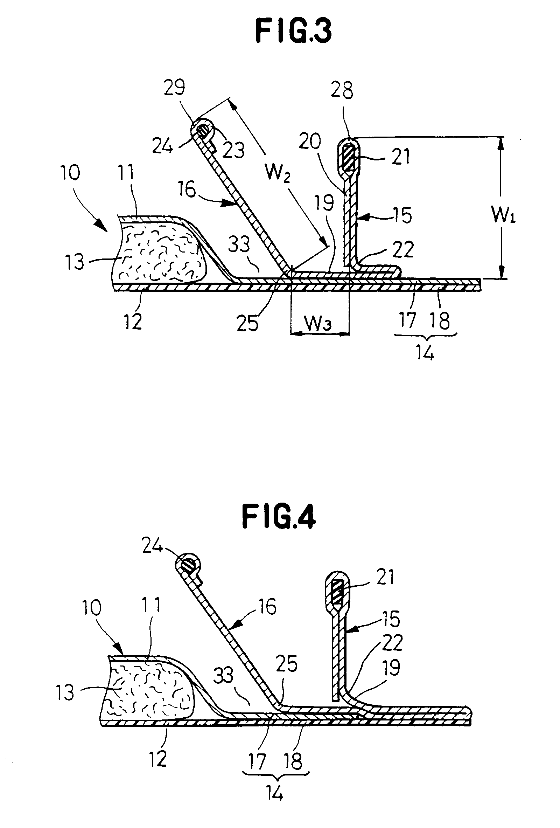

Bei einem alternativen Ausführungsbeispiel, wie es in Fig. 4 gezeigt ist, weicht jede der Grundklappen 14 einen Abschnitt 17 einer inneren Lage und einen Abschnitt 18 einer äußeren Lage auf, deren Breite größer ist als diejenige des Abschnitts 17. Mit anderen Worten, die Grund- oder Basisklappe 14 ist so ausgebildet, daß der genannte Abschnitt 18 sich von der Außenseitenkante des Abschnitts 17 nach außen erstreckt. Die Lage 19 ist teilweise auf eine solche Verlängerung des Abschnitts 18 aufgeklebt, entlang welcher der genannte Abschnitt 17 nicht auf der Oberseite dieses Abschnitts liegt und bildet dadurch die ersten und zweiten Klappen 15,16. Dieses Ausführungsbeispiel ist insbesondere vorteilhaft, weil für die innere Lage 11 eine Materialerspanis erzielt wird, da die innere Lage 11 eine Breite haben darf, die geringer ist als diejenige der äußeren Lage 12. Es kann jegliche Leckage von Körperflüssigkeit, die andernfalls jenseits der äußeren Seitenkanten der jeweiligen Basisklappen 14 auftreten könnte, vermieden werden, selbst wenn die Flüssigkeit in den flüssigkeitsdurchlässigen Abschnitt 17 der inneren Lage eindringt oder diffundiert. Dies wird deshalb verhindert, weil die flüssigkeitshemmende Lage 19, welche die ersten und zweiten Klappen 15,16 bildet, an den Abschnitt 18 der flüssigkeitsundurchlässigen äußeren Lage angeklebt wird ohne daß die flüssigkeitsdurchlässige innere Lage dazwischengelegt ist.

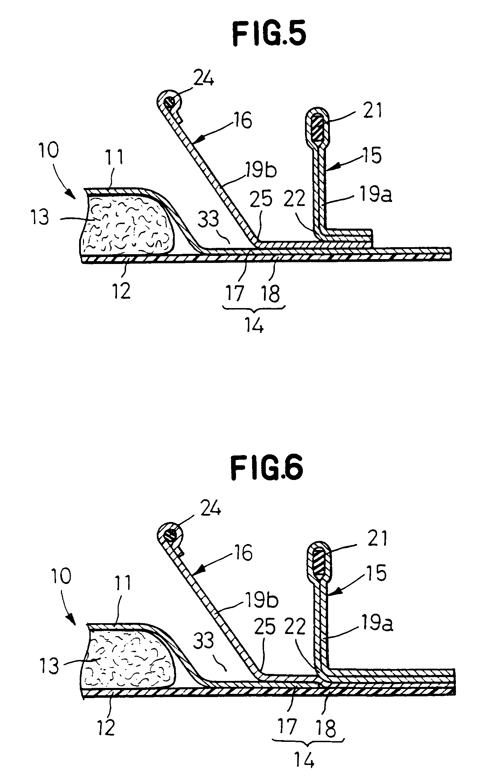

Die ersten und zweiten Klappen werden bei einem weiteren Ausführungsbeispiel gemäß den Figuren 5 und 6 aus unterschiedlichen Lagen 19a und 19b gebildet. Eine Wasserdruckfestigkeit (Flüssigkeitsundurchlässigkeit) der ersten Klappe 15 ist so eingestellt, daß sie einen höheren Wert aufweist als diejenige der zweiten Klappe 16. Außer einem solchen Merkmal ist der Aufbau der ersten und zweiten Klappen 15,16 und der Grundklappen 14 in diesem Ausführungsbeispiel im wesentlichen derselbe wie bei dem Ausführungsbeispiel gemäß der Figuren 3 und 4.

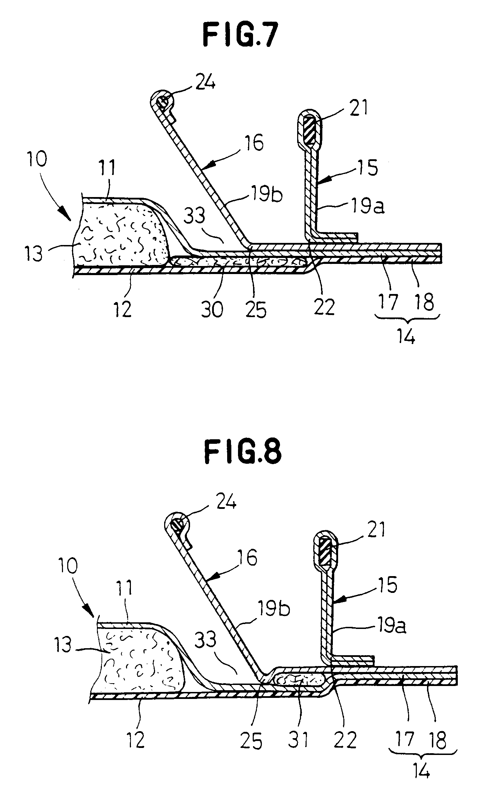

In dem in Fig. 7 dargestellten Ausführungsbeispiel ist zwischen den Abschnitten 17 und 18 der inneren und äußeren Lagen eine relativ dünne hochflexible Sauglauge 30 angeordnet, wobei sie sich zwischen der äußeren Seitenkante des Kerns 13 und der Wurzelkante 22 der zugeordneten ersten Klappe 15 erstreckt. In dem in Fig. 8 dargestellten Ausführungsbeispiel ist eine relativ dünne, hochflexible Sauglage 31 zwischen dem Abschnitt 17 der inneren Lage und der Lage 19b vorgesehen,so daß sie sich zwischen den Wurzelkanten 22,25 der ersten und zweiten Klappe 15,16 erstreckt. Diese Ausführungsformen sind so ausgelegt, daß sie eine Flüssigkeitssaugfähigkeit zumindest zwischen den Wurzelkanten 22,25 besitzen, so daß jede Menge an Körperflüssigkeit, welche zwischen den ersten und zweiten Klappen 15,16 stehen, selbst wenn dies eine längere Zeit wäre, von den jeweiligen Lagen 30,31 absorbiert werden kann.

Wie aus den Figuren 1 und 2 zu erkennen ist, sind Bandbefestiger mit druckempfindlichem Klebstoff an den jeweiligen Grundklappen 14 auf der Rückseite der Windel 10 vorgesehen. Die Windel 10 kann am Körper des Trägers mittels der freien Befestigungsenden der jeweiligen Bandbefestiger 32 auf der äußeren Lage 12 festgelegt werden.

Die innere Lage 11 ist aus einem Material wie ein Faserfilm oder porösem Kunststoffilm und die äußere Lage 12 aus einem Material wie Kunststoffilm oder einer Laminatlage aus einem Film und einem Faservlies hergestellt. Es versteht sich, daß eine solche Laminatlage mit der Faservliesschicht nach außen gewandt verwendet wird. Der Kunststoffilm, der als Material für die Außenlage 12 geeignet ist, weist vorzugsweise luftsperrende Eigenschaften auf. Der Kern 13 besitzt einen mattenartigen Körper, der aus flockiger Pulpe besteht, die mit einem superabsorbierendem Polymerpuder gemischt sein kann und mit einer flüssigkeitsdurchlässigen Lage zumindest auf der Ober- und der Unterseiterseite bedeckt ist. Hinsichtlich des Materials, aus dem der Kern 13 gebildet ist, ist der Kern 13 halbsteif. Es versteht sich, daß der Kern 13 ziemlich eng an die innere Lage 11 und/oder die äußere Lage 12 mit einem Klebstoff angeklebt sein kann. Die elastischen Teile 21 bzw. 24 weisen faden- oder bandförmigen Gummi, bandförmigen Kunststoffschaum oder einen Plastikfilm mit elastischer Dehnfähigkeit unter Wärmebehandlung auf. Die ersten und zweiten Klappen 15,16 werden aus einem wasserabstoßenden Faservlies hergestellt. Es ist jedoch nicht immer wesentlich, daß die zweiten Klappen 16 aus einem flüssigkeitsfesten Material bestehen, so lange als diese zweiten Klappen 16 ordnungsgemäß funktionieren, um eine mögliche Leckage fester Ausscheidungen aufzuhalten, da die zweiten Klappen 16, insbesondere über ihrer inneren Oberfläche surfactant behandelt worden sind und dadurch hydrophile Eigenschaften erhalten haben. Die absorbierenden Lagen 30,31 weisen dünne Streifen aus flockiger Pulpe auf, die mit einem superabsorbierenden Polymerpulver gemischt sein kann.

Wenn die Windel 10, die die oben beschriebene Konstruktion aufweist, an einen Träger angelegt wird, richten sich die zweiten Klappen 15,16 entlang der jeweiligen Wurzelkanten 22,25 aufgrund der elastischen Zusammenziehfähigkeit der jeweiligen elastischen Teile 21,24, die von den jeweiligen freien Kanten 28,29 umhüllt sind, auf. Beide, die erste und die zweite Klappe 15,16 schneiden mit ihren Wurzelkanten 22,25 die zugeordnete Basisklappen 14 oder biegen sich relativ zu der zugeordneten Basisklappe 14, so daß sie einen umgekehrten T-förmigen Querschnitt bilden mit der Folge, daß die elastische Zusammenziehfähigkeit der jeweiligen elastischen Teile 21,24 im wesentlichen daran gehindert ist, über die jeweiligen Wurzelkanten 22,25 auf die äußeren Seiten der jeweiligen Basisklappen übertragen zu werden. Mit anderen Worten, die Gegenwart der Wurzelkanten 22,25 minimiert das Phänomen, daß die elastische Zusammenziehfähigkeit der elastischen Teile 21,24 undmittelbar auf die Außenseiten der jeweiligen Grundklappen 14 übertragen wird, was dazu führt, daß die Außenseiten faltig werden können.

## Patentansprüche

1. Wegwerfkleidungsstück mit einer flüssigkeitsdurchlässigen inneren Lage (11), einer flüssigkeitsdurchlässigen äußeren Lage (12), einem im wesentlichen zwischen zwei Beinöffnungen angeordneten Saugkern (13), mit einem Paar Basisklappen (14), die sich im wesentlichen um die Beinöffnungen des Kleidungsstücks herum seitlich nach außen erstrecken und mit einem ersten Paar Klappen (15), die sich von dem vorderen Bereich des Kleidungsstücks bis zu seinem rückwärtigen Bereich erstrecken und zumindest jeweils an ihrer freien Randkante mit einem elastischen Teil versehen sind, wodurch die Klappe im angelegten Zustand des Kleidungsstücks aufgerichtet ist, **dadurch gekennzeichnet,** daß jeweils auf der dem Saugkern (13) zugewandten Seite der Klappe (15) in einem Abstand eine weitere zweite aufrichtbare Klappe (16) angeordnet ist,
- das erste Klappenpaar (15) Wurzelkanten (22) aufweist, die sich auf den seitlich einander gegenüberliegenden Seiten des Kerns (13) erstrecken, um sich unter ihrer eigenen elastischen Zusammenzieheigenschaft aufzurichten, wobei das zweite Klappenpaar (16) seine Wurzelkanten (25) innen im Abstand von den jeweiligen Wurzelkanten (22) des ersten Klappenpaares aufweist, so daß sie sich unter der Einwirkung ihres eigenen elastischen Zusammenziehpotentials aufrichten können,
- jede zweite Klappe (16) eine Zugspannung aufweist, die geringer ist als die Zugspannung der äußeren ersten Klappe (15)
- die Breite jeder zweiten Klappe (16) gemessen von ihrer Wurzelkante (25) bis zur freien Randkante (24) größer ist als die Breite der ersten Klappe (15), gemessen in der gleichen Weise
- und die Steifigkeit jeder zweiten Klappe (16) geringer ist als die Steifigkeit jeder ersten Klappe (15).

2. Wegwerfkleidungsstück nach Anspruch 1, wobei die ersten und zweiten Klappenpaare (15,16) entlang jeweiliger Wurzelränder von der zugeordneten Basisklappe (14) abzweigen und die Oberfläche jeweiliger Basisklappen (14) zwischen den benachbarten Wurzelkanten (22,25) der zugeordneten ersten und zweiten Klappen (15,16) mit Abschnitten (19) der jeweiligen zweiten Klappen (16) bedeckt sind und wobei eine flüssigkeitsabsorbierende Lage (30,31) zwischen den Abschnitten (19) und der zweiten Klappe (16) und Abschnitten der Basisklappen (14) angeordnet sind und die Abschnitte (19) der zweiten Klappen (16) jeweils unterlegen.

3. Wegwerfkleidungsstück nach Anspruch 1, wobei die ersten und zweiten Klappenpaare (15,16) an ihren in Längsrichtung einander gegenüberliegenden Enden mit dem ersten Paar der Klappen (15) nach innen oder außen geklappt und die Klappen des zweiten Klappenpaares (16) nach innen geklappt befestigt sind.

## Claims

1. A disposable garment, with an inner layer (11) permeable to liquids, an outer layer (12) impermeable to liquids, an absorbent core (13) arranged substantially between two leg openings, with a pair of base flaps (14) which extend laterally outwards substantially around the leg openings of the garment and are provided with a first pair of flaps (15) which extend from the front region of the garment up to its rear region and are provided at least at their respective free edges with an elastic part, whereby the flap is erected when the garment is worn, characterized in that a further erectile flap (16) is arranged spaced from the side of the flap (15) facing the absorbent core (13),
- the first flap pair (15) has root edges (22) which extend on the laterally opposite sides of the core (13), in order to erect under their own elastic contractile characteristic, wherein the second flap pair (16) has its root edges (25) spaced inside the respective root edges (22) of the first flap pair, so that they can erect under the action of their own capability to contract,
- each second flap (16) has a tension which is smaller than the tension in the outer first flap (15),
- the width of each second flap (16) measured from its root edge (25) to its free edge (24) is greater than the width of the first flap (15) measured in like manner,
- and the stiffness of each second flap (16) is smaller than the stiffness of each first flap (15).

2. A disposable garment according to claim 1, wherein the first and second flap pairs (15, 16) branch off from the associated base flap (14) along respective root edges and the surface of the respective base flap (14) between the adjacent root edges (22, 25) of the associated first and second flaps (15, 16) are covered by sections (19) of the respective second flaps (16) and wherein liquid absorbent layers (30, 31) are arranged between the sections (19) of the second flaps (16) and sections of the base flaps (14) and lie under the respective sections (19) of the second flaps (16).

3. A disposable garment according to claim 1, wherein the first and second flap pairs (15, 16) are fixed at their ends opposite one another in the longitudinal direction with the first pair of flaps (15) turned in or out and the flaps of the second flap pair (16) turned in.

## Revendications

1. Vêtement jetable comprenant une couche intérieure perméable aux liquides (11), une couche extérieure imperméable aux liquides (12), un corps central absorbant (13) disposé, essentiellement, entre deux ouvertures de passage des jambes, deux volants ou rabats de base (14) s'étendant latéralement vers l'extérieur, pour l'essentiel, autour des ouvertures de passage des jambes du vêtement, et deux premiers rabats (15) partant de la zone avant du vêtement et se prolongeant jusque dans sa zone arrière, et pourvus chacun au moins d'un élément élastique sur sa lisière libre, ce qui a pour effet de redresser lesdits rabats lorsque le vêtement est enfilé, caractérisé en ce que de chaque côté des rabats (15) orienté vers le corps absorbant (13), on prévoit un deuxième rabat plus large et redressable (16),
- les deux premiers rabats (15) présentent des racines (22) qui s'étendent sur les côtés du corps absorbant (13) en relation d'opposition latérale mutuelle, pour se redresser sous l'effet de leur contractibilité élastique propre, les racines (25) de la seconde paire de rabats (16) étant espacées, à l'intérieur, des racines respectives (22) de la première paire de rabats, ce qui leur permet de se redresser sous l'effet de leur potentiel de contraction élastique propre,
- chaque second rabat (16) présente une contrainte de tension inférieure à celle du premier rabat extérieur (15);
- la largeur de chaque second rabat (16), mesurée de sa racine (25) jusqu'à sa lisière libre (24), est plus grande que la largeur du premier rabat (15), mesurée de la même façon;
- et la rigidité de chaque second rabat (16) est inférieure à celle de chaque premier rabat (15).

2. Vêtement jetable selon la revendication 1, dans lequel les première et seconde paires de rabats (15, 16) se détachent du rabat de base associé (14), au niveau des racines, et la surface de rabats de base (14) comprise entre les racines voisines (22, 25) des premiers et seconds rabats associés (15, 16) est recouverte par des portions (19) des seconds rabats (16), et dans lequel une couche (30, 31) absorbant les liquides est prévue entre les portions (19) et le second rabat (16) et des portions des rabats de base (14), et assurent un doublage des portions (19) des seconds rabats (16).

3. Vêtement jetable selon la revendication 1, dans lequel les première et seconde paires de rabats (15, 16) sont rabattues vers l'intérieur ou l'extérieur à leurs extrémités longitudinalement opposées, avec la première paire de rabats (15), et les rabats de la seconde paire de rabats (16) sont fixés après avoir été rabattus vers l'intérieur.
